# EUROPEAN PATENT APPLICATION

(11) **EP 4 321 620 A1**
(43) Date of publication of application: **14.02.2024**
(21) Application number: 22783792.9
(22) Date of filing: 11.02.2022
(51) Int. Cl.: C12N 11/18, C12N 11/06, C12P 19/24, C12P 19/18, C12P 19/16, C12P 19/02

(54) **METHOD FOR PRODUCING TAGATOSE BY IMMOBILIZING MULTIPLE ENZYMES BY USING ARTIFICIAL OIL BODY**

(30) Priority: 07.04.2021 CN 202110370762
(71) Applicant: TIANJIN YEAHE BIOTECHNOLOGY CO., LTD, Tianjin, 300308 (CN)
(72) Inventor: MA, Yanhe, Tianjin 300308 (CN); SHI, Ting, Tianjin 300308 (CN); HAN, Pingping, Tianjin 300308 (CN); LI, Yunjie, Tianjin 300308 (CN)
(74) Representative: Pharma Patents International AG
(86) International application number: PCT/CN2022/076063
(87) International publication number: WO 2022/213721

(57) **Abstract**

Provided are the immobilization of multiple enzymes on the basis of an artificial oil body and an application thereof in the preparation of tagatose. Specifically, an artificial oil body is used to mix an expressed fusion protein of target protease-oil body protein with an oil body, which then undergoes an ultrasonic treatment; the fusion protein is anchored to the surface of the oil body by means of the specific hydrophobicity of a human protein to form an artificial oil body containing the target protease, so that the purification and immobilization of enzymes can be completed simultaneously. The immobilized multiple enzymes that can be used for tagatose production utilize an artificial oil body as an immobilized enzyme substrate, which significantly improves the stability of the immobilized enzymes, reduces the production cost of the current enzymatic preparation of tagatose, and has a simple preparation process.

## Description

### Field of the Invention

The present invention relates to the field of genetic engineering, and specifically relates to expression of enzyme or protein and a method for preparing immobilized multi-enzymes to produce tagatose.

### Background of the Invention

Currently, a mainstream method for producing tagatose comprises steps of, for example, galactose isomerization, desalination, decolorization, separation, concentration, and crystallization to obtain pure tagatose. However, this method has several disadvantages. With this method, the galactose cannot be completely converted into tagatose, and thus the final product is a mixture of galactose and tagatose, resulting in a low conversion rate and the need of a complex tagatose separation process. While the cost for tagatose separation is high, the galactose as a raw material is not at a low price, both contributing to a high cost for tagatose production. (Rhimi M, Aghajari N, Juy M, Chouayekh H, Maguin E, Haser R, Bejar S: Rational design of Bacillus stearothermophilus US1001-arabinose isomerase: Potential applications for d-tagatose production. Biochim. 2009, 91:650-653. Oh H-J, Kim H-J, Oh D-K: Increase in d-tagatose production rate by site-directed mutagenesis of 1-arabinose isomerase from Geobacillus thermodenitrificans. Biotechnol. Lett. 2006, 28:145-149. Bosshart A, Hee CS, Bechtold M, Schirmer T, Panke S:Directed divergent evolution of a thermostable D-tagatose epimerase towards improved activity for two hexose substrates. ChemBioChem 2015,16:592-601.) Both the CJ corporation in Korea and the Tianjin Institute of Industrial Biotechnology, Chinese Academy of Sciences have developed a new route for producing tagatose with multiple enzymes *in vitro* (WO2018004310A1, CN109790524A, CN107988286A, and CN109666620A). With this route, starch, maltodextrin, or sucrose can be used as a raw material to produce tagatose through enzyme-based catalytic reactions in multiple steps. It represents a fundamental change from the existing isomerization-based tagatose production process. However, this new route involves multiple enzyme molecules which require a plurality of steps for extraction and purification before they can be used in the catalytic reactions, resulting in a high cost for enzyme preparation. Moreover, the biological enzymes are water-soluble molecules and thus can hardly be recovered and reused after the completion of the catalytic reactions, resulting in a waste of enzymes. These factors lead to the high production cost for the new route of tagatose production. Therefore, there is an urgent need to develop a method for immobilizing multiple enzymes, which can immobilize the multiple enzymes in the new route of tagatose production, so that the enzymes can be recycled and the production cost can be lowered.

Oil bodies are important lipid storage organelles in plant seeds, especially those of oil plant species. They have a structural model consisted of an elastic sphere or ellipsoid formed from a liquid triacylglycerol matrix encapsulated by a monomolecular layer of phospholipids with oil body-bound proteins embedded therein. They show significant physical and chemical stabilities. Oil bodies have been extensively developed and widely applied in a variety of biotechnological fields including protein expression, purification, and immobilization, encapsulation of probiotics and bioactive substances, and alternatives of emulsifiers. Artificial oil body refer to the stable oil body formed by mixing the main components of triacylglycerol, phospholipid, and oleosin in a certain ratio *in vitro* and allowing them to assemble. A process of preparing artificial oil body is as follows: first, constructing a recombinant expression vector for an oleosin and a target protein; then efficiently expressing the recombinant fusion protein in the form of an inclusion body in *Escherichia coli*; then disrupting the bacterial cell, centrifuging, and collecting precipitate; and finally forming artificial oil body together with phospholipid and triacylglycerol.

By constructing artificial oil body, the multiple steps including protein purification, immobilization, and renaturation can be achieved simultaneously in one simple step, avoiding the protein purification step in a traditional protein immobilization process, thereby saving time for and reducing the cost of protein immobilization. At present, the recombinant cellulase (Chiang C J, Chen P T, Yeh C Y, et al. A useful method integrating production and immobilization of recombinant cellulose. Appl. Microbiol. Biotechnol. 2013, 97 (20 ): 9185-9192), lipase (CN102321693A), psicose epimerase (Immobilization of Clostridium cellulolyticumd-Psicose 3-Epimerase on Artificial Oil Bodies, J. Agric. Food Chem. 2014, 62, 28, 6771-6776) and the like have been immobilized using this method.

In summary, the immobilization of multiple enzymes with artificial oil body will be a research focus. It is still necessary to find or develop a strategy to apply artificial oil body in co-immobilization of multiple enzymes, so as to establish a new technology for producing tagatose based on catalysis with multiple enzymes immobilized with artificial oil body. This technology shall improve the catalytic efficiency of the cascade catalytic reactions and reduce the production cost with enzyme-based catalysis.

### Summary of the Invention

Although use of artificial oil body in immobilizing single enzyme has the advantages such as a low cost, a simple process, and significantly improved stabilities and reusability of the immobilized enzyme, major deficiencies exist when the artificial oil body-immobilized single enzyme is used in a system of tagatose production based on enzyme catalytic cascade reactions. The present inventors have found from research that the tagatose production pathway which is based on catalysis with multiple enzymes involves five different enzyme molecules. When the five enzymes are immobilized separately, mixed and used in catalytic reactions, there is a significant problem with the substrate/product mass transfer. An upstream product can hardly be captured by a downstream immobilized enzyme quickly, which significantly reduces the catalytic efficiency of the catalytic cascade reactions. In view of this, the present inventors propose co-immobilization of the five enzymes for catalytic reaction, which greatly improves the reaction effects.

Thus, an object of the present invention is to address the major problem in substrate/product mass transfer in catalytic cascade reactions with immobilized multi-enzymes, and improve the catalytic efficiency of the catalytic cascade reactions with immobilized multi-enzymes. An artificial oil body is used to immobilize the five enzymes in the tagatose production process. With the artificial oil body, and the five enzyme molecules mixed in a specific ratio, purification and immobilization of the multiple enzymes are achieved simultaneously. Use of the artificial oil body co-immobilized multi-enzymes avoids the mass transfer problem caused by enzymes immobilized in a separate immobilization step, thereby improving the efficiency of cascade catalysis based on multiple enzymes. It also enhances the stability and the recycling effect of the multi-enzymes system. The present invention provides a novel enzyme immobilization technology for the tagatose production, which reduces the production cost with the current enzymatic method for producing tagatose and has a simple preparation process.

The present invention provides artificial oil body-immobilized enzyme, characterized in that the artificial oil body-immobilized enzyme is obtained by mixing an α-glucan phosphorylase-oleosin fusion protein, a phosphoglucomutase-oleosin fusion protein, a phosphoglucose isomerase-oleosin fusion protein, a tagatose 6-phosphate 4-epimerase-oleosin fusion protein, and a tagatose 6-phosphate phosphatase-oleosin fusion protein, mixing with an oil body and a phospholipid, ultrasonicating, centrifuging and collecting an upper layer of material, which is the artificial oil body-immobilized enzyme.

In an embodiment, the fusion proteins are respectively obtained by constructing an expression vector with an oleosin gene and a gene for each enzyme using a genetic engineering method, respectively transforming into recombinant bacteria, and expressing.

In an embodiment, the oleosin gene is a sesame oleosin gene, a soybean oleosin gene, or a peanut oleosin gene. More specifically, the oleosin gene has a nucleotide sequence as shown in SEQ ID NO.1.

In an embodiment, the enzymes are thermostable, which are a thermostable α-glucan phosphorylase, a thermostable phosphoglucomutase, a thermostable phosphoglucose isomerase, a thermostable tagatose 6-phosphate 4-epimerase, and a thermostable tagatose 6-phosphate phosphatase. Compared with heat-liable enzymes which are suited to room temperature, thermostable enzymes are advantageous during strain inactivation. The latter enables heat treatment for strain inactivation after fermentation, as the enzymes related to tagatose production can maintain their activities during the heat treatment. As a result, the thermostable enzymes enable use of a mixture of inactivated strains to produce tagatose, which is more suitable for industrial applications.

Specifically, the thermostable α-glucan phosphorylase refers to the enzyme having the function to turn starch into glucose-1-phosphate (G1P) through phosphorylation at 40°C or above, 45°C or above, 50°C or above, 55°C or above, 60°C or above, 65°C or above, 70°C or above, 75°C or above, or 80°C or above. Further preferably, the thermostable α-glucan phosphorylase is derived from a thermophilic microorganism, for example, *Geobacillus kaustophilus, Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus*; or the thermostable α-glucan phosphorylase has an amino acid sequence which is at least 70%, preferably at least 80%, more preferably at least 90%, or most preferably at least 95% identical to that of the thermostable α-glucan phosphorylase derived from the thermophilic microorganisms. More preferably, the thermostable α-glucan phosphorylase is derived from *Thermotoga maritima.*

Specifically, the thermostable phosphoglucomutase refers to the enzyme having the function to turn glucose-1-phosphate (G1P) into glucose-6-phosphate (G6P) through change of position at 40°C or above, 45°C or above, 50°C or above, 55°C or above, 60°C or above, 65°C or above, 70°C or above, 75°C or above, or 80°C or above. Further preferably, the thermostable phosphoglucomutase is derived from a thermophilic microorganism, for example, *Geobacillus kaustophilus*, *Geobacillus stearothermophilus, Thermotoga maritima*, *Pseudothermotoga thermarum, Thermococcus kodakarensis, Archaeoglobus fulgidus*, *Thermoanaerobacter indiensis, Dictyoglomus thermophilum, Caldicellulosiruptor kronotskyensis, Clostridium thermocellum, Caldilinea aerophila, Pyrococcus furiosus*, *Thermus thermophilus, Methanothermobacter marburgensis,* or *Archaeoglobus profundus*; or the thermostable phosphoglucomutase has an amino acid sequence which is at least 70%, preferably at least 80%, more preferably at least 90%, or most preferably at least 95% identical to that of the thermostable phosphoglucomutase derived from the thermophilic microorganism. More preferably, the thermostable phosphoglucomutase is derived from *Pyrococcus furiosus.*

Specifically, the thermostable phosphoglucose isomerase refers to the enzyme having the function to turn glucose-6-phosphate (G6P) into fructose-6-phosphate (F6P) through change of position at 40°C or above, 45°C or above, 50°C or above, 55°C or above, 60°C or above, 65°C or above, 70°C or above, 75°C or above, or 80°C or above. Further preferably, the thermostable phosphoglucose isomerase is derived from a thermophilic microorganism, for example, *Geobacillus kaustophilus*, *Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus*, *Thermus thermophilus, Methanothermobacter marburgensis,* or *Archaeoglobus profundus*; or the thermostable phosphoglucose isomerase has an amino acid sequence which is at least 70%, preferably at least 80%, more preferably at least 90%, or most preferably at least 95% identical to that of the thermostable phosphoglucose isomerase derived from the thermophilic microorganism. More preferably, the thermostable phosphoglucose isomerase is derived from *Thermus thermophilus.*

Specifically, the thermostable tagatose 6-phosphate 4-epimerase refers to the enzyme having the function to turn fructose-6-phosphate (F6P) into tagatose-6-phosphate (T6P) through isomerization at 40°C or above, 45°C or above, 50°C or above, 55°C or above, 60°C or above, 65°C or above, 70°C or above, 75°C or above, or 80°C or above. Further preferably, the thermostable tagatose 6-phosphate 4-epimerase is derived from a thermophilic microorganism, for example, *Geobacillus kaustophilus*, *Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus*; or the thermostable tagatose 6-phosphate 4-epimerase has an amino acid sequence which is at least 70%, preferably at least 80%, more preferably at least 90%, or most preferably at least 95% identical to that of the thermostable tagatose 6-phosphate 4-epimerase derived from the thermophilic microorganism. More preferably, the tagatose 6-phosphate 4-epimerase is derived from *Caldicellulosiruptor kronotskyensis.*

Specifically, the tagatose 6-phosphate phosphatase refers to the enzyme having the function to turn tagatose-6-phosphate (T6P) into tagatose as a product by removing the phosphate group at 40°C or above, 45°C or above, 50°C or above, 55°C or above, 60°C or above, 65°C or above, 70°C or above, 75°C or above, or 80°C or above. Further preferably, the tagatose 6-phosphate phosphatase is derived from a thermophilic microorganism, for example, *Geobacillus kaustophilus*, *Geobacillus stearothermophilus, Thermotoga maritima*, *Pseudothermotoga thermarum, Thermococcus kodakarensis, Archaeoglobus fulgidus*, *Thermoanaerobacter indiensis, Dictyoglomus thermophilum, Caldicellulosiruptor kronotskyensis, Clostridium thermocellum, Caldilinea aerophila, Pyrococcus furiosus*, *Thermus thermophilus, Methanothermobacter marburgensis,* or *Archaeoglobus profundus*; or the tagatose 6-phosphate phosphatase has an amino acid sequence which is at least 70%, preferably at least 80%, more preferably at least 90%, or most preferably at least 95% identical to that of the tagatose 6-phosphate phosphatase derived from the thermophilic microorganism. More preferably, the tagatose 6-phosphate phosphatase is derived from *Archaeoglobus fulgidus.*

In a preferred embodiment, the α-glucan phosphorylase-oleosin fusion protein, the phosphoglucomutase-oleosin fusion protein, the phosphoglucose isomerase-oleosin fusion protein, the tagatose 6-phosphate 4-epimerase-oleosin fusion protein, and the tagatose 6-phosphate phosphatase-oleosin fusion protein are mixed in a mass ratio of (1-2) : (1-2) : (1-2) : (2-4) : (2-4).

Preferably, the oil body is a triglyceride or an animal or vegetable oil containing a triglyceride; the phospholipid is, for example, derived from an animal phospholipid, specifically, lecithin, cephalin, or cardiolipin or the like.

In a preferred embodiment, the fusion proteins all together are mixed with triglyceride in a mass ratio of (0.5-3) : 1, triglyceride and lecithin are mixed in a mass ratio of 100 : 1, and then subjected to ultrasonic treatment.

In a preferred embodiment, the ultrasonicating is carried out at a power of 20-30% for 5-15 min; the centrifuging is carried out at 8,000 to 12,000 rpm/min for 5-15 min.

The present invention also provides use of the artificial oil body-immobilized enzyme in preparation of tagatose.

In an embodiment, tagatose is prepared by using starch or a starch derivative as a raw material, and carrying out enzyme-based catalytic conversions with the artificial oil body-immobilized multi-enzymes. More specifically, the steps include taking 50-150 g/L of starch or starch derivative, an 80-120 mM HEPES buffer at pH 6.0-7.0, 8-12 mM inorganic phosphate, 3-7 mM divalent magnesium ions, 0.3-0.7 mM zinc ions or manganese ions, 3-7 U/ml of debranching enzyme, and 1-5 mg/ml of the artificial oil body-immobilized multi-enzymes; carrying out enzyme-based catalytic conversion reaction at 40-70°C, and collecting the tagatose produced.

Further, solid-liquid separation is carried out after the reaction is completed, and the artificial oil body-immobilized multi-enzymes are collected and reused for preparation of tagatose. For example, the artificial oil body-immobilized multi-enzymes can be recycled for 2-20 times.

The present invention uses the artificial oil body co-immobilized multi-enzymes, and thus avoids the mass transfer problem caused by use of immobilized single enzymes in multi-enzymes-based cascade catalytic reactions. It achieves purification and immobilization of multiple enzymes simultaneously, and simplifies the process of preparing immobilized enzymes. The artificial oil body as a matrix for enzyme immobilization not only has a low cost, but also improves the stabilities of the immobilized enzymes. After the reactions for tagatose production are completed, the artificial oil body-immobilized multi-enzymes can be recycled after simple treatment, thereby lowering the production cost.

### Brief Description of the Drawings

Figure 1 shows a pattern for plasmid construction.

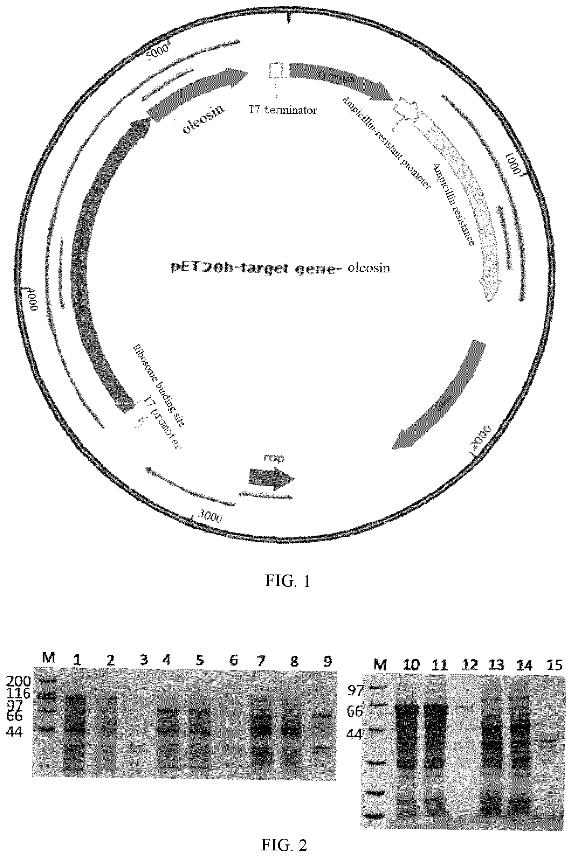

Figure 2 shows the SDS-PAGE electrophoresis analysis of the fusion proteins, in which Lane M represents the marker protein; Lanes 1-3 relate to the glucan phosphorylase, representing the total proteins of cells, the supernatant after ultrasonication, and the precipitate after ultrasonication respectively; Lanes 4-6 relates to the phosphoglucomutase, representing the total proteins of cells, the supernatant after ultrasonication, and the precipitate after ultrasonication respectively; Lanes 7-9 relate to the phosphoglucose isomerase, representing the total proteins of cells, the supernatant after ultrasonication, and the precipitate after ultrasonication respectively; Lanes 10-12 relate to the tagatose 6-phosphate 4-epimerase, representing the total proteins of cells, the supernatant after ultrasonication, and the precipitate after ultrasonication respectively; and Lane 13-15 relate to the tagatose 6-phosphate phosphatase, representing the total proteins of cells, the supernatant after ultrasonication, and the precipitate after ultrasonication respectively.
Figure 3 shows the process for preparing artificial oil body-immobilized enzyme.
Figure 4 shows the determination of the activity of the artificial oil body-immobilized glucan phosphorylase.
Figure 5 shows the determination of the activity of the artificial oil body-immobilized phosphoglucomutase.
Figure 6 shows the determination of the activity of the artificial oil body-immobilized phosphoglucose isomerase.
Figure 7 shows the determination of the activity of the artificial oil body-immobilized tagatose 6-phosphate 4-epimerase.
Figure 8 shows the determination of the activity of the artificial oil body-immobilized tagatose 6-phosphate phosphatase.
Figure 9 shows the tagatose production through reactions with a mixture of artificial oil body-immobilized single enzymes.
Figure 10 shows the determination of the concentration of tagatose produced with the artificial oil body-immobilized multi-enzymes.
Figure 11 shows the recycling of the artificial oil body-immobilized multi-enzymes.

### Detailed Description of the Invention

The present invention will be further described through embodiments and examples below. A person skilled in the art can, referring to the contents of the present invention, appropriately modify the parameters for implementation of the processes. Noted that all similar replacements or modifications are apparent to a person skilled in the art and are considered within the scope of the present invention. It is apparent that relevant personnel can modify or appropriately change and combine the methods and applications described herein to implement or apply the technology of the present invention without departing from the content, spirit, and scope of the present invention.

### Example 1 Construction of Expression Vectors and Expression of Fusion Proteins

### 1. Construction of Expression Vectors

Genetic recombination technologies were used to construct expression vectors comprising the genes of the glucan phosphorylase-oleosin fusion protein, the phosphoglucomutase-oleosin fusion protein, the phosphoglucose isomerase-oleosin fusion protein, the tagatose 6-phosphate 4-epimerase-oleosin fusion protein, and the tagatose 6-phosphate phosphatase-oleosin fusion protein.

In this example, the gene encoding the first 140 amino acids of the sesame-derived oleosin (NCBI Reference Sequence: XP_011076526.1) was codon optimized (SEQ ID NO. 1), and incorporated into the pET20b vector (with cleavage sites of NdeI and XhoI) through genetic synthesis to obtain the pET20b-oleosin. Subsequently, the five genes, that is, the genes of the α-glucan phosphorylase derived from *Thermotoga maritima*, with gene No. TM1168 on KEGG; the phosphoglucomutase derived from *Pyrococcus furiosus*, with gene No. PF0588 on KEGG; the phosphoglucose isomerase derived from *Thermus thermophilus*, with gene No. TTHA0277 on KEGG; the tagatose 6-phosphate epimerase derived from *Caldicellulosiruptor kronotskyensis*, with the gene encoded enzyme No. Calkro_0564 on KEGG; and the tagatose 6-phosphate phosphatase, with gene No. AF_0444 on KEGG, were obtained through genetic synthesis. They were cloned into the pET20b-oleosin vectors to obtain the respective expression vectors of pET20b-TmαGP-oleosin, pET20b-pfuPGM-oleosin, pET20b-TtcPGI-oleosin, pET20b-CkTPE-oleosin, and pET20b-AfTPP-oleosin. The schematic diagram for the plasmid construction was shown in Figure 1.

### 2. Expression of Fusion Proteins

All the above plasmids were transformed into the *Escherichia coli* (*E. coli*) expression strain BL21 (DE3) (Invitrogen, Carlsbad, CA). Single clones were picked for culture for fermentation, and when the OD₆₀₀ = 0.8-1.0, they were induced with isopropyl-β-D-thiogalactoside (IPTG, 200 mM/L) to express a large amount of fusion proteins. The bacteria-containing solutions were collected, and centrifuged at 6,000 rpm/min for 10 min. The bacteria were washed with a 0.9% sodium chloride solution and collected. They were disrupted through ultrasonication (at a power of 50% for 10 min), and centrifuged. The precipitates were collected to obtain the fusion proteins. The obtained fusion proteins were analyzed by sodium dodecyl sulfonate polyacrylamide gel electrophoresis. The results were shown in Figure 2.

### Example 2 Artificial oil body-Immobilized Single Enzymes and Determination of Their Activities

### 1. Artificial oil body-immobilized glucan phosphorylase

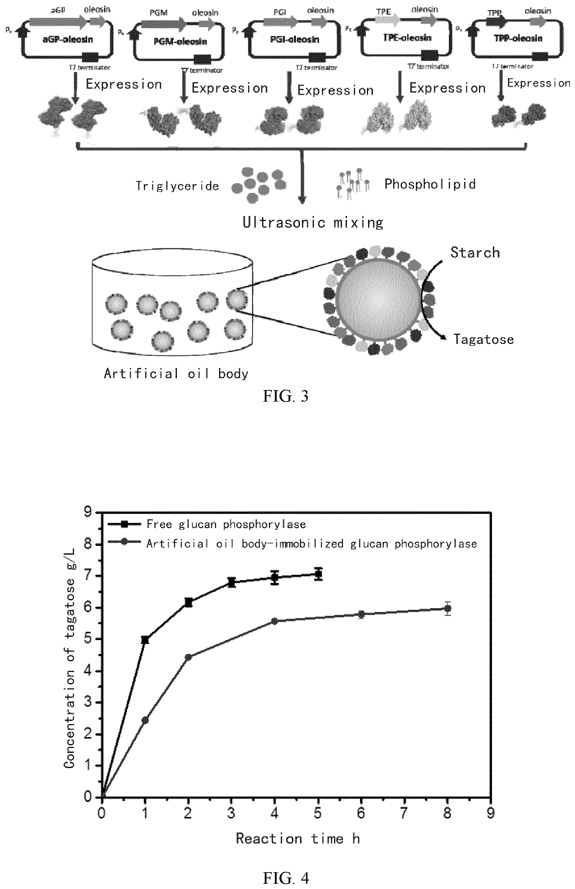

The artificial oil body-immobilized glucan phosphorylase of the present invention was prepared according to the process shown in Figure 3.

The glucan phosphorylase-oleosin fusion protein (100 mg) prepared in Example 1 was added to a test tube. Then 75 mg of triglycerides and 750 ug of lecithin were added. The sample was ultrasonicated (at a power of 20-30%) or 10 minutes, and then centrifuged at 10,000 rpm/min. The white substance of the upper layer, which was the artificial oil body-immobilized glucan phosphorylase, was collected.
**2.** Artificial oil body-immobilized phosphoglucomutase: the preparation method comprised the same steps as the above point 1, except that the immobilized enzyme was phosphoglucomutase instead of glucan phosphorylase.
**3.** Artificial oil body-immobilized phosphoglucose isomerase: the preparation method comprised the same steps as the above point 1, except that the immobilized enzyme was phosphoglucose isomerase instead of glucan phosphorylase.
**4.** Artificial oil body-immobilized tagatose 6-phosphate 4-epimerase: the preparation method comprised the same steps as the above point 1, except that the immobilized enzyme was tagatose 6-phosphate 4-epimerase instead of glucan phosphorylase.
**5.** Artificial oil body-immobilized tagatose 6-phosphate phosphatase: the preparation method comprised the same steps as the above point 1, except that the immobilized enzyme was tagatose 6-phosphate phosphatase instead of glucan phosphorylase.
**6.** Determination of activities of artificial oil body-immobilized single enzymes

Each of the above immobilized single enzymes prepared in this Example was used to prepare tagatose respectively using the following method.

Ten (10) g/L of starch, a 100 mM HEPES buffer at pH 6.5, 40 mM inorganic phosphate, 5 mM divalent magnesium ions, 0.5 mM zinc ions or manganese ions, 1 U/ml of debranching enzyme, 0.1 g/L of glucan phosphorylase, 0.1 g/L of phosphoglucomutase, 0.1 g/L of phosphoglucose isomerase, 0.2 g/L of tagatose 6-phosphate 4-epimerase and 0.2 g/L of tagatose 6-phosphate phosphatase were taken. Reactions were carried out at 70°C. The concentration of tagatose was determined by high performance liquid chromatography (HPLC). To determine the activities of the above immobilized single enzymes of this Example, at a time, one of the immobilized single enzymes prepared in this Example was used to replace its corresponding free enzyme to determine its activity.

When the immobilized glucan phosphorylase prepared in this Example was used in the above reactions, 2.5 g/L of tagatose was produced after 1 hour of reaction, and 5.5 g/L of tagatose was produced after 8 hours of reaction when the reaction equilibrium was reached. When the corresponding free enzyme was used, 5.0 g/L of tagatose was produced after 1 hour of reaction, and 7.0 g/L of tagatose was produced after 5 hours of reaction when the reaction equilibrium was reached. Compared with the corresponding free enzyme, the artificial oil body-immobilized glucan phosphorylase had 50% initial relative enzymatic activity (Figure 4).

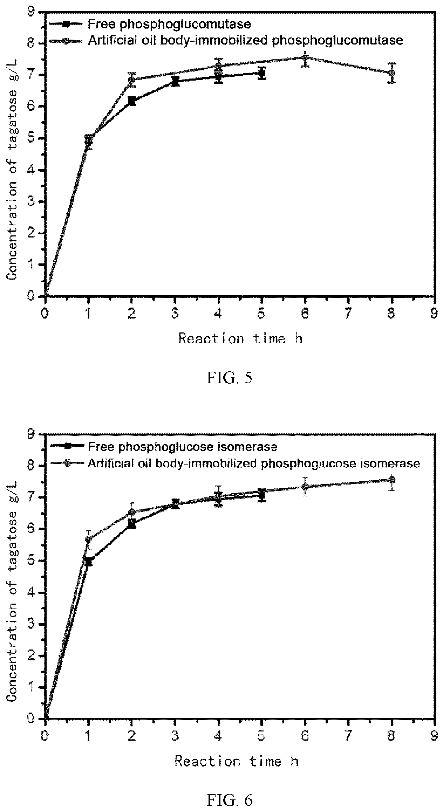

When the immobilized phosphoglucomutase prepared in this Example was used in the above reactions, 5.0 g/L of tagatose was produced after 1 hour of reaction, and 7.4 g/L of tagatose was produced after 6 hours of reaction when the reaction equilibrium was reached. When the corresponding free enzyme was used, 5.0 g/L of tagatose was produced after 1 hour of reaction, and 7.0 g/L of tagatose was produced after 5 hours of reaction when the reaction equilibrium was reached. Compared with the corresponding non-immobilized enzyme, the artificial oil body-immobilized phosphoglucomutase had 100% initial relative enzymatic activity (Figure 5).

When the immobilized phosphoglucose isomerase prepared in this Example was used in the above reactions, 5.7 g/L of tagatose was produced after 1 hour of reaction, and 7.5 g/L of tagatose was produced after 8 hours of reaction when the reaction equilibrium was reached. When the corresponding free enzyme was used, 5.0 g/L of tagatose was produced after 1 hour of reaction, and 7.0 g/L of tagatose was produced after 5 hours of reaction when the reaction equilibrium was reached. Compared with the corresponding non-immobilized enzyme, the artificial oil body-immobilized phosphoglucose isomerase had 114% initial relative enzymatic activity (Figure 6).

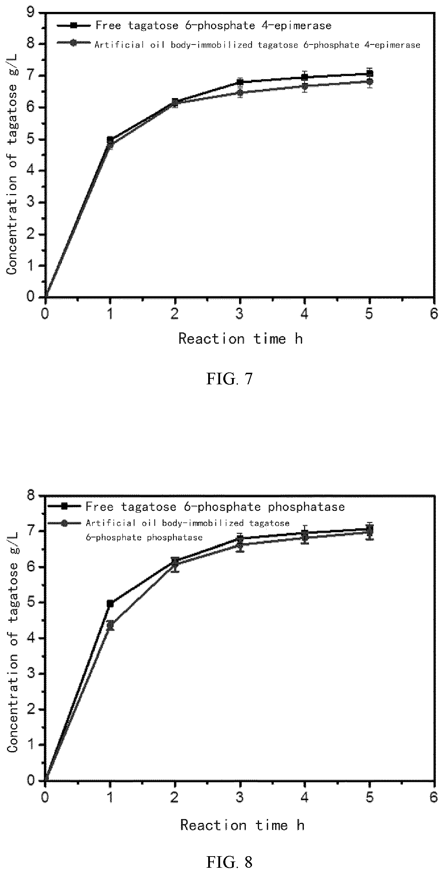

When the immobilized tagatose 6-phosphate 4-epimerase prepared in this Example was used in the above reactions, 5.5 g/L of tagatose was produced after 1 hour of reaction, and 6.6 g/L of tagatose was produced after 5 hours of reaction when the reaction equilibrium was reached. When the corresponding free enzyme was used, 5.0 g/L of tagatose was produced after 1 hour of reaction, and 7.0 g/L of tagatose was produced after 5 hours of reaction when the reaction equilibrium was reached. Compared with the corresponding free enzyme, the artificial oil body-immobilized tagatose 6-phosphate 4-epimerase had 110% initial relative enzymatic activity (Figure 7).

When the artificial oil body-immobilized tagatose 6-phosphate phosphatase prepared in this Example was used in the above reactions, 4.5 g/L of tagatose was produced after 1 hour of reaction, and 6.8 g/L of tagatose was produced after 5 hours of reaction when the reaction equilibrium was reached. When the corresponding free enzyme was used, 5.0 g/L of tagatose was produced after 1 hour of reaction, and 7.0 g/L of tagatose was produced after 5 hours of reaction when the reaction equilibrium was reached. Compared with the corresponding free enzyme, the artificial oil body-immobilized tagatose 6-phosphate phosphatase had 90% initial relative enzymatic activity (Figure 8).

### Example 3 Tagatose production through reactions with a mixture of artificial oil body-immobilized single enzymes.

All the immobilized single enzymes prepared in Example 2 were used to prepare tagatose using the following method.

A hundred (100) g/L of starch, a 100 mM HEPES buffer at pH 6.5, 40 mM inorganic phosphate, 5 mM divalent magnesium ions, 0.5 mM zinc ions or manganese ions, and 5 U/ml of debranching enzyme were taken. The immobilized single enzymes prepared in Example 2 were mixed, including 0.1 g/L of glucan phosphorylase, 0.1 g/L of phosphoglucomutase, 0.1 g/L of phosphoglucose isomerase, 0.2 g/L of tagatose 6-phosphate 4-epimerase and 0.2 g/L of tagatose 6-phosphate phosphatase. Reactions were carried out at 70°C, and the concentration of tagatose was determined by HPLC.

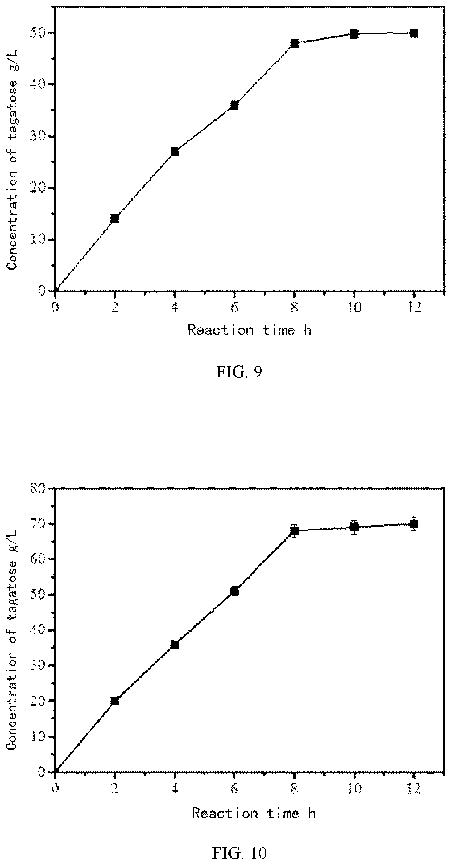

The results were shown in Figure 9. After 2 hours of reaction, tagatose was produced at a concentration of 14 g/L. After 12 hours of reaction, the reactions for tagatose production tended to reach equilibrium, producing tagatose at a concentration of 50 g/L.

### Example 4 Tagatose Production with artificial oil body-Immobilized Multi-enzymes

The five fusion proteins (100 mg) prepared in Example 1 were added to a test tube in a mass ratio of glucan phosphorylase-oleosin fusion protein : phosphoglucomutase-oleosin fusion protein : phosphoglucose isomerase-oleosin fusion protein : tagatose 6-phosphate 4-epimerase-oleosin fusion protein : tagatose 6-phosphate phosphatase-oleosin fusion protein = 1.5 : 1.5 : 1.5 : 2 : 2. Then 75 mg of triglycerides and 750 ug of lecithin were added. The sample was ultrasonicated (at a power of 20-30%) for 10 min, and then centrifuged at 10,000 r/min. The white substance of the upper layer, which was the artificial oil body-immobilized multi-enzymes, was collected.

The immobilized multi-enzymes provided by this Example were used to prepare tagatose using the following method.

A hundred (100) g/L of starch, a 100 mM HEPES buffer at pH 6.5, 40 mM inorganic phosphate, 5 mM divalent magnesium ions, 0.5 mM zinc ions or manganese ions, 5 U/ml of debranching enzyme, and the above immobilized enzymes provided by this Example (or non-immobilized enzymes) including 0.1 g/L of glucan phosphorylase, 0.1 g/L of phosphoglucomutase, 0.1 g/L of phosphoglucose isomerase, 0.2 g/L of tagatose 6-phosphate 4-epimerase and 0.2 g/L of tagatose 6-phosphate phosphatase were taken. Reactions were carried out at 70°C. The concentration of tagatose was determined by HPLC.

As shown in Figure 10, the HPLC analysis indicated that after 2 hours of reaction, tagatose was produced at a concentration of 21 g/L; and after 12 hours of reaction, the reactions for tagatose production reached equilibrium, producing tagatose at a concentration of 70 g/L. Compared with Example 2, Example 3 had a higher initial rate for tagatose production and a higher tagatose production concentration.

### Example 5 Recycling of Artificial oil body-Immobilized Multi-enzymes

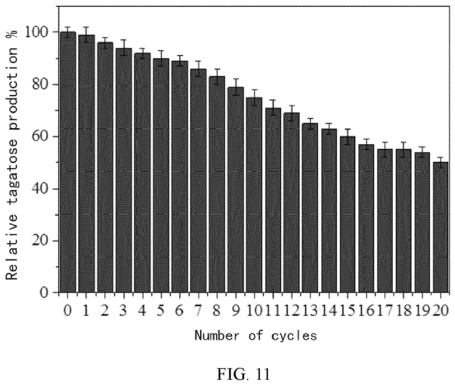

After tagatose was prepared with the method provided by Example 3, solid-liquid separation was carried out. The immobilized multi-enzymes were collected and reused for preparation of tagatose. The concentration of tagatose produced in each cycle was determined by HPLC. The results were expressed as relative tagatose production concentration. The concentration of tagatose produced in the first cycle of reactions was set as 100%. As shown by results in Figure 11, the recycled artificial oil body-immobilized multi-enzymes still had 50% relative enzymatic activity after 20 cycles.

Although the present invention has been described in detail with general description, embodiments, and experiments above, it is obvious to a person skilled in the art that some modifications or improvements can be made based on the present invention. Therefore, these modifications or improvements made without departing from the spirit of the present invention are within the claimed scope of the present invention.

## Claims

1. An artificial oil body-immobilized enzyme, **characterized in that** the artificial oil body-immobilized enzyme is obtained by mixing an α-glucan phosphorylase-oleosin fusion protein, a phosphoglucomutase-oleosin fusion protein, a phosphoglucose isomerase-oleosin fusion protein, a tagatose 6-phosphate 4-epimerase-oleosin fusion protein, and a tagatose 6-phosphate phosphatase-oleosin fusion protein, mixing with an oil body and a phospholipid, ultrasonicating, centrifuging and collecting an upper layer of material, which is the artificial oil body-immobilized enzyme.

2. The artificial oil body-immobilized enzyme according to claim 1, **characterized in that** the fusion proteins are respectively obtained by constructing an expression vector with an oleosin gene and a gene for each enzyme using a genetic engineering method, respectively transforming into recombinant bacteria, and expressing.

3. The artificial oil body-immobilized enzyme according to claim 1, **characterized in that** the oleosin gene is a sesame oleosin gene, a soybean oleosin gene, or a peanut oleosin gene.

4. The artificial oil body-immobilized enzyme according to claim 3, **characterized in that** the oleosin gene has a nucleotide sequence as shown in SEQ ID NO.1.

5. The artificial oil body-immobilized enzyme according to claim 1, **characterized in that** the enzymes encoded by each enzyme gene are thermostable enzymes.

6. The artificial oil body-immobilized enzyme according to claim 5, **characterized in that** the enzyme genes are derived from *Geobacillus kaustophilus, Geobacillus stearothermophilus, Thermotoga maritima, Pseudothermotoga thermarum, Thermococcus kodakarensis, Archaeoglobus fulgidus, Thermoanaerobacter indiensis, Dictyoglomus thermophilum, Caldicellulosiruptor kronotskyensis, Clostridium thermocellum, Caldilinea aerophila, Pyrococcus furiosus, Thermus thermophilus, Methanothermobacter marburgensis,* or *Archaeoglobus profundus.*

7. The artificial oil body-immobilized enzyme according to claim 1, **characterized in that** the α-glucan phosphorylase-oleosin fusion protein, the phosphoglucomutase-oleosin fusion protein, the phosphoglucose isomerase-oleosin fusion protein, the tagatose 6-phosphate 4-epimerase-oleosin fusion protein, and the tagatose 6-phosphate phosphatase-oleosin fusion protein are mixed in a mass ratio of (1-2) : (1-2) : (1-2) : (2-4) : (2-4).

8. The artificial oil body-immobilized enzyme according to claim 1, **characterized in that** the oil body is a triglyceride or an animal or vegetable oil containing a triglyceride; the phospholipid is lecithin, cephalin, or cardiolipin.

9. The artificial oil body-immobilized enzyme according to claim 8, **characterized in that** the fusion proteins all together are mixed with triglyceride in a mass ratio of (0.5-3) : 1, triglyceride and lecithin are mixed in a mass ratio of 100 : 1, and then subjected to ultrasonic treatment.

10. The artificial oil body-immobilized enzyme according to claim 1, **characterized in that** the ultrasonicating is carried out at a power of 20-30% for 5-15 min; the centrifuging is carried out at 8,000 to 12,000 rpm/min for 5-15 min.

11. Use of the artificial oil body-immobilized enzyme according to any of claims 1-10 in preparation of tagatose.

12. The use according to claim 11, **characterized in that** the tagatose is prepared by using starch or a starch derivative as a raw material, and carrying out enzyme-based catalytic conversion with the artificial oil body-immobilized multi-enzymes.

13. The use according to claim 12, **characterized in that** specific steps comprise taking 50-150 g/L of starch or starch derivative, an 80-120 mM HEPES buffer at pH 6.0-7.0, 8-12 mM inorganic phosphate, 3-7 mM divalent magnesium ions, 0.3-0.7 mM zinc ions or manganese ions, 3-7 U/ml of debranching enzyme, and 1-5 mg/ml of the artificial oil body-immobilized multi-enzymes, carrying out enzyme-based catalytic conversion reaction at 40-70°C, and collecting the tagatose produced.

14. The use according to claim 13, **characterized in that** solid-liquid separation is carried out after the reaction is completed, and the artificial oil body-immobilized multi-enzymes are collected and recycled for preparation of tagatose.
